# EUROPEAN PATENT SPECIFICATION

(11) **EP 3 466 837 B1**
(45) Date of publication and mention of the grant of the patent: **24.03.2021**
(21) Application number: 17802439.4
(22) Date of filing: 31.03.2017
(51) Int. Cl.: B65D 83/08, A47K 7/00, B65D 77/04

(54) **WET SHEET PACKAGE**
VERPACKUNG FÜR FEUCHTTÜCHER
EMBALLAGE POUR FEUILLES HUMIDES

(30) Priority: 26.05.2016 JP 2016105515
(43) Date of publication of application: 10.04.2019
(73) Proprietor: Shinohara, Yuji, Niihama-shi, Ehime 792-0893 (JP); Glide Enterprise Inc., Shibuya-ku, Tokyo 150-0002 (JP)
(72) Inventor: SHINOHARA, Yuji, Ehime 7920893 (JP)
(74) Representative: Lefevre-Groboillot, David André
(86) International application number: PCT/JP2017/013862
(87) International publication number: WO 2017/203838

(56) References cited:
- DE-A1- 2 432 578
- JP-A- S5 077 183
- JP-A- 2003 020 081
- JP-A- 2009 078 828
- JP-A- 2010 042 846
- JP-A- 2010 285 176
- JP-A- 2016 041 593
- JP-A- 2016 041 593
- JP-U- S 623 472
- JP-U- S 623 472
- US-A- 3 265 241
- US-B1- 6 349 525

## Description

### Field of the Invention

0001 The present invention relates to wet-sheet packaging in which wet sheets, such as wet wipes or facemasks for face packing, are packaged so that each wet sheet can be taken out from the packaging.

### Background of the Invention

0002 Wet-sheet packaging that contains, in layers inside an outer bag, wet sheets such as facemasks for face packing is configured such that (1) the outer bag has gas-barrier capability; (2) the outer surface of the outer bag is provided with a wet-sheet take-out opening and a covering that can freely be opened and closed; (3) when a user wants to take out a wet sheet, the user opens the covering and takes out the wet sheet through the take-out opening; and (4) the user, after having taken out the wet sheet, firmly closes the covering over the take-out opening.

0003 In the prior-art wet-sheet packaging, if the packaging is to contain large-size wet sheets such as facemasks, the wet-sheet packaging is configured such that: (1) each wet sheet is folded into three parts in the widthwise direction to form a band shape; (2) the band-shaped wet sheet is folded in its lengthwise direction into two or three sections to form a substantially rectangular-shape folded body of a desired size; (3) multiple folded facemasks are stacked in the vertical direction (thickness direction) so as to form a layered body; and (4) the layered body is accommodated inside an outer bag. (Patent Document 1)

### Prior-Art Document

### Patent Document

0004
Patent Document 1: Japanese Unexamined Patent Application Publication No. 2010-28517
Patent Document 2: Japanese Unexamined Patent Application Publication No. 2016-41593
Patent Document 3: Japanese Unexamined Utility Model (Registration) Application Publication No. S62-3472

### Summary of the Invention

### Technical Problems to be Solved

0005 As is achieved by the prior-art wet-sheet packaging, if wet sheets are packaged in such a way that they have been folded into a desired size, the entire wet-sheet packaging can be relatively small, even when the wet sheets to be packaged are somewhat large; for example, when the wet sheets are facemasks.

0006 However, when facemasks are packaged as wet sheets, each facemask is taken out from an outer bag in a folded state, and then must be spread out by a user before the facemask is applied on the user's face. Therefore, the prior-art facemask has a disadvantage that a user experiences trouble in handling the prior-art facemask, in that the user needs to go through a process that requires that the facemask be taken out from an outer bag, spread out, and then applied on the user's face.

0007 In particular, the facemask has a problem that the user experiences difficulties in spreading out the facemask after it has been taken out from a package, because the facemask includes openings at prescribed positions, such as openings for eyes, a cut-out part for a nose, and an opening for a mouth, and the portions of the facemask opposite to each other in a folded state tend to become adhered to each other because much of the cosmetic liquid for face packing has permeated into the facemasks.

0008 In view of the above problems associated with the prior-art wet-sheet packaging, the present invention aims to provide wet-sheet packaging that eliminates the troubles that a user encounters in spreading out a wet sheet after the user has taken it out from the packaging. As a result, the convenience of the use of the wet-sheet packaging is improved.

### Solution to the Problems

0009 The present invention provides wet-sheet packaging that includes an outer bag that includes, at its upper side, an outer take-out opening that is freely opened and closed by a closure means, and a layered body of wet sheets accommodated in the outer bag, with each wet sheet in a folded state, and wherein the layered body is accommodated so that an edge of the upper surface-side of the topmost wet sheet can be grasped, enabling the wet sheet to be spread out by a user when the wet sheet is being taken out of the packaging.

0010 Each of the wet sheets is folded into a band shape to be layered, with the edge of the folded wet sheet disposed at the middle part of the folded sheet in the width direction so as to face the lengthwise direction. The folded wet sheet is configured such that each of the wet sheets is folded into a zigzag shape.

0011 The layered body further includes a layer-top part that faces the outer take-out opening, and layer-side parts that stretch from both sides of the layer-top part to each of the bottom edges of the layered body. The outer bag may include an inner container that has shape-retention capability and that accommodates the layered body. The wet-sheet packaging may include an inner sheet by which an opening of the inner container is closed and within which an inner take-out opening corresponding to the outer take-out opening is formed.

0012 The inner container may include a projection that protrudes from the container's bottom to support the underside of the layer-top part that faces the outer take-out opening of the layered body, and recessed parts that are formed at both sides of the projection and that accommodate each of the layer-side parts that stretch from the layer-top part to each of the bottom edges of the layered body. The outer take-out opening may be a size that allows a user to insert two fingers from each of his/her left and right hands into the opening to grasp two portions of the edge.

0013 The wet sheet may be a facemask for face packing, in which case the wet sheet is permeated with a cosmetic liquid. The facemask may be folded in reverse directions at multiple fold lines disposed in the lengthwise direction, and the edge may be at the outer periphery of the upper side of the facemask.

### Advantageous Effects of the Invention

0014 The present invention's wet-sheet packaging provides advantages over the prior art in that it eliminates the troubles that a user encounters in spreading out a wet sheet after the user has taken it out from the packaging, which improves the convenience in use of the wet sheet.

### Brief Descriptions of the Drawings

0015
Fig. 1 is a perspective view of the open wet-sheet packaging according to the First Embodiment of the present invention.
Fig. 2 is a sectional view of open wet-sheet packaging according to the First Embodiment of the present invention.
Fig. 3 is a plan view of open wet-sheet packaging according to the First Embodiment of the present invention.
Fig. 4(a) and Fig. 4(b) are enlarged views of a peripheral portion of an inner container of the wet-sheet packaging according to the First Embodiment of the present invention.
Fig. 5 is an exploded perspective view of the inner sheet, inner container, and receiving plate of the wet-sheet packaging according to the First Embodiment of the present invention.
Figs. 6(a) to 6(d) show the folding and layering of wet sheets according to the First Embodiment of the present invention.
Fig. 7 is a sectional side view that shows a wet sheet being taken out from the wet-sheet packaging according to the First Embodiment of the present invention.
Fig. 8 is a sectional front view that shows a wet sheet being taken out from the wet-sheet packaging according to the First Embodiment of the present invention.
Figs. 9(a) to 9(c) are plan views of the inner sheet according to the Second Embodiment of the present invention.
Fig. 10 is a plan view of a facemask according to the Third Embodiment of the present invention.
Fig. 11 consists of three sectional views of an inner container in which a layered body is accommodated according to the Fourth Embodiment of the present invention.
Fig. 12 consists of two sectional view of an inner container in which a layered body is accommodated according to the Fifth Embodiment of the present invention.
Fig. 13(a) is a plan view of facemask, Fig. 13(b) is a perspective view of a facemask in a folded state, and Fig. 13(c) is a sectional view of a facemask that is accommodated in an inner container according to the Sixth Embodiment of the present invention.
Fig. 14 is a sectional view of an inner container in which a layered body is accommodated according to the Seventh Embodiment of the present invention.
Fig. 15 is a perspective view of the inner container and the inner lid thereof according to the Seventh Embodiment of the present invention.

### Descriptions of the Embodiments

0016 The present invention will be specifically explained below according to the Embodiments with reference to the attached drawings.

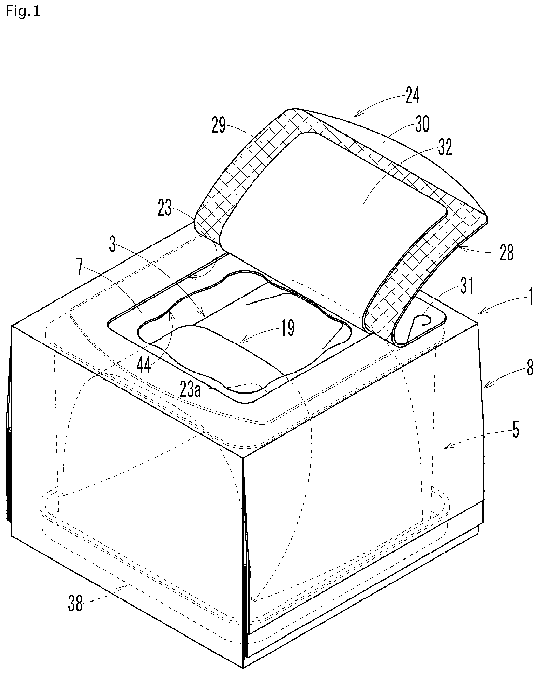

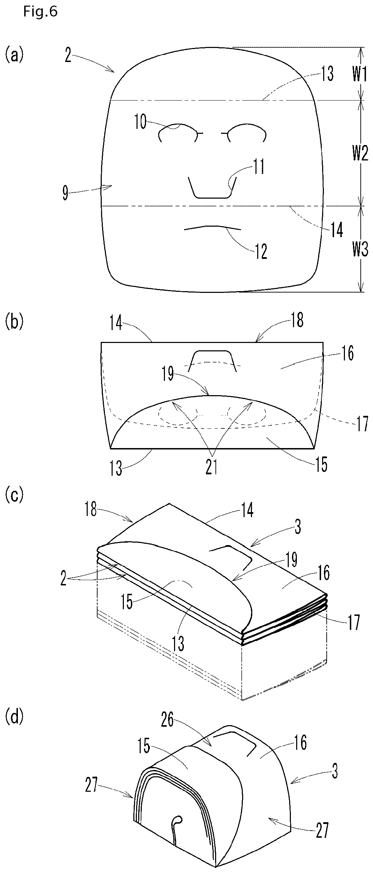

0017 Figs. 1 to 8 show the First Embodiment of the wet-sheet packaging 1 of the present invention. As shown in Figs. 1 to 3, the wet-sheet packaging 1 includes (1) a layered body 3 in which multiple wet sheets such as facemasks 2 are stacked (see Fig. 6), and (2) a permeating liquid 4 such as a cosmetic liquid that is to permeate into the wet sheets 2 of the layered body 3. The wet-sheet packaging 1 is configured such that (1) the layered body 3 and the permeating liquid 4 are put inside an inner container 5, (2) the upper opening 6 of the inner container 5 is firmly closed by an inner sheet 7, and (3) an outer bag 8 wraps the entire outside of the inner container in order that the entire packaging can be sealed shut. The above configuration results in the wet-sheet packaging being airtight and having the shape of a cuboid or a hexahedron that is approximately a cube.

0018 The outer bag 8 is configured by using a packaging method such as pillow packaging to form into a bag an exterior material such as a composite film or a compound sheet that has gas-barrier capability. Each side of the outer bag 8 is formed into an approximately square shape. As shown in Figs. 1 to 3, the outer surface of the outer bag 8 is provided with (1) an outer take-out opening 23, and (2) a closure means 24 that is freely opened and closed to firmly close the outer take-out opening 23. The outer take-out opening 23 is formed into a substantially rectangular shape having a size that is appropriate for facemasks 2 to be taken out.

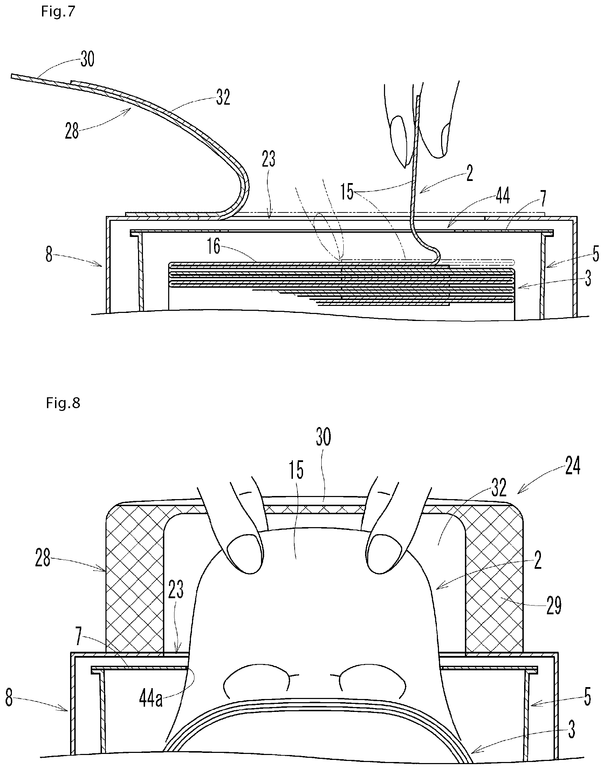

0019 A user takes the following steps to use facemasks 2 that are contained in the wet-sheet packaging 1: (1) open the outer take-out opening 23 of the outer bag 8 by using the closure means 24; (2) insert two fingers from each of his/her left and right hands into the inner container 5 through the outer take-out opening 23; (3) take out the topmost facemask 2 of the layered body 3 while spreading the taken-out facemask 2, as shown in Figs. 7 and 8; and (4) use the closure means 24 to firmly close the outer bag 8.

0020 As shown in Fig. 6(a), the facemask 2 consists of a sheet 9 that is prepared by cutting a sheet material, such as a nonwoven fabric, into a shape that corresponds to a human face, and from which sheet 9 parts are cut out for eyes 10, a nose 11, and a mouth 12. Also, openings for eyes may be provided instead of the cut-out parts for eyes 10, and an opening for a mouth may be provided instead of the cut-out part for a mouth 12.

0021 In this Embodiment, the facemask 2 is presented as an example of a wet sheet, but other types of sheets, including wet wipes, may be used as wet sheets. If the wet sheets are facemasks 2, a cosmetic liquid for face packing is used as the permeating liquid 4. If the wet sheets are wet wipes or other types of sheets, a liquid that is suitable for each such types of sheet should be used.

0022 The layered body 3 of a facemask 2 is configured such that (1) the sheet 9 is folded at fold lines 13, 14, that are substantially parallel to each other in a Z shape (zigzag shape) so that the facemask 2 is formed as a folded facemask 18, whose upper portion 15, middle portion 16, and lower portion 17 overlap each other to form the folded facemask 18, which forms a rectangle shape, as shown in Fig. 6(b). Multiple folded facemasks 18 are vertically layered to form a layered body 3 as shown in Fig. 6(c).

0023 As shown in Fig. 6(a), the fold lines 13, 14 are approximately parallel to each other, and are configured such that (1) the width W1 of the upper portion 15 is approximately one-half of the width W2 of the middle portion 16; and (2) the width W3 of the lower portion 17 is almost the same as or slightly smaller than the width W2 of the middle portion 16. The sheet 9 is formed into a band-shaped folded facemask 18 in such a way that (1) the upper portion 15 is folded along the fold line 13 to be placed over the middle portion 16; and (2) the lower portion 17 is folded along the fold line 14 to be placed under the middle portion 16.

0024 Accordingly, as shown in Figs. 7 and 8, the folded facemask 18 is capable of being unfolded so that the facemask 2 is spread in such a way that, when a user takes out the facemask 2 from the inner container 5, the user grasps, with two fingers from each of his/her right and left hands, the two pullable portions 21 shown in Fig. 6(b), which are located on the two sides of the approximately central portion of an edge 19 of the upper portion 15, and upwardly pulls the pullable portions 21. Also, the edge 19, which includes the pullable portions 21, is at the outer periphery on the upper side of the facemask 2.

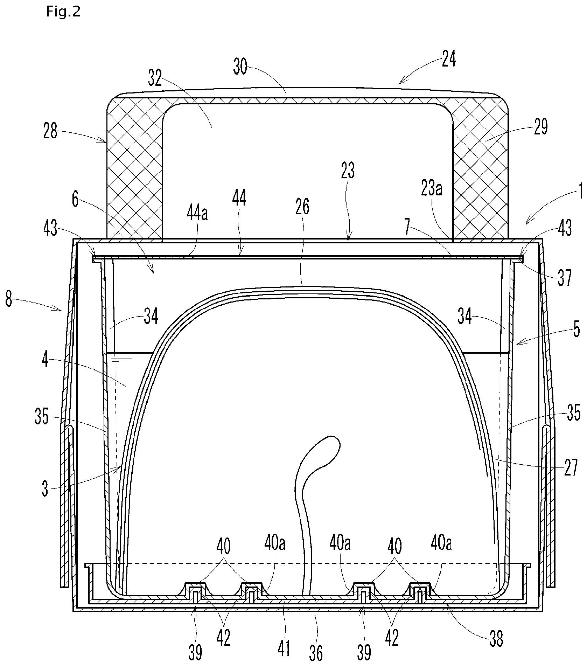

0025 As shown in Fig. 6(d), the layered body 3 is accommodated inside the inner container 5 in such a way that multiple folded facemasks 18 in a layered state are bent in their lengthwise direction into an approximately inverted U-shape. Therefore, in the layered body 3, as shown in Fig. 2, the edge 19 of the upper portion 15 of the folded facemask 18 includes, in the lengthwise direction of the folded facemask 18, a layer-top part 26 in an upward-arc shape that is opposite the outer take-out opening 23, and layer-side parts 27 that are disposed to stretch from both sides of the layer-top part 26 to each of the bottom edges of the layered body 3.

0026 The closure means 24 is made of a closure sheet 28. As shown in Figs. 1 to 3, the closure sheet 28 includes (1) an adhesive part 29 that is formed so as to extend around the entire outer periphery of the outer take-out opening 23, (2) a tab part 30 that is provided outside of the adhesive part 29, and (3) a separation-prevention part 31 that is provided to the other side of the tab part 30 of the closure means 24. The adhesive part 29 of the closure sheet 28 other than the part corresponding to the separation-prevention part 31 is separably attached to the upper surface of the outer bag 8. In addition, a cut-out piece 32 that corresponds to the outer take-out opening 23 of the outer bag 8 is removed from the closure sheet 28.

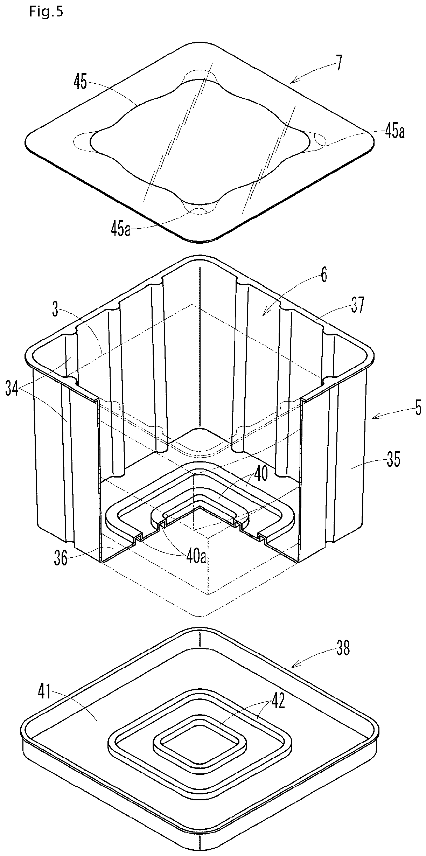

0027 The inner container 5 is made of a relatively thin plastic that is thick enough to allow the inner container 5 to retain its original shape. As shown in Figs. 2 and 5, the inner container 5 includes a tapered peripheral wall 35 that spreads outward in the upward direction, a bottom 36 that is formed integrally with the lower end of the peripheral wall 35, and an upper peripheral edge 37 that is bent outward from the upper end of the peripheral wall 35 as a flange. The inner container 5 also includes at its upper side an opening 6, and is packaged by the outer bag 8 by being placed on a receiving plate 38 under the inner container 5. A positioning means 39 is provided between the inner container 5 and the receiving plate 38.

0028 The peripheral wall 35 is provided with multiple vertical reinforcing parts 34, and the bottom 36 is provided with projections 40 that face the layered body 3. The reinforcing parts 34 are provided to secure the vertical rigidity of the peripheral wall 35. Each of the reinforcing parts 34 is formed such that it protrudes inward into the inner container 5 and such that each reinforcing part 34 has a groove shape. However, the reinforcing part may be formed such that it instead protrudes outward. The projection 40 is formed to be a planar quadrilateral shape that is peripherally continuous. The inner container 5 includes multiple projections 40 that are concentrically disposed. Each of the projections 40 protrudes upward from the bottom 36 of the inner container 5. The back side of each of the projections 40 forms a positioning recess 40a.

0029 The receiving plate 38 is configured to have substantially the same size as the outer side of the upper peripheral edge 37 of the inner container 5, so that after the inner container 5 has been placed in the outer bag 8, the upper and bottom edges of the wet-sheet packaging 1 have substantially the same dimensions. The bottom part 41 of the receiving plate 38 is provided with positioning projections 42 that enter the corresponding positioning recesses 40a of the inner container 5 from the underside of the positioning recesses 40a. The positioning means 39 consists of a positioning recess 40a and a positioning projection 42.

0030 A sheet material such as a composite film having gas-barrier capability is used for an inner sheet 7, which is fixed at fixed parts 43 to the entirety of the upper peripheral edge 37 of the inner container 5 by using an adhesion material such as a heat-sealing film so that the opening 6 of the inner container 5 is firmly closed. Also, the inner sheet 7 is fixed such that virtually its entire surface becomes approximately flat in a stretched state.

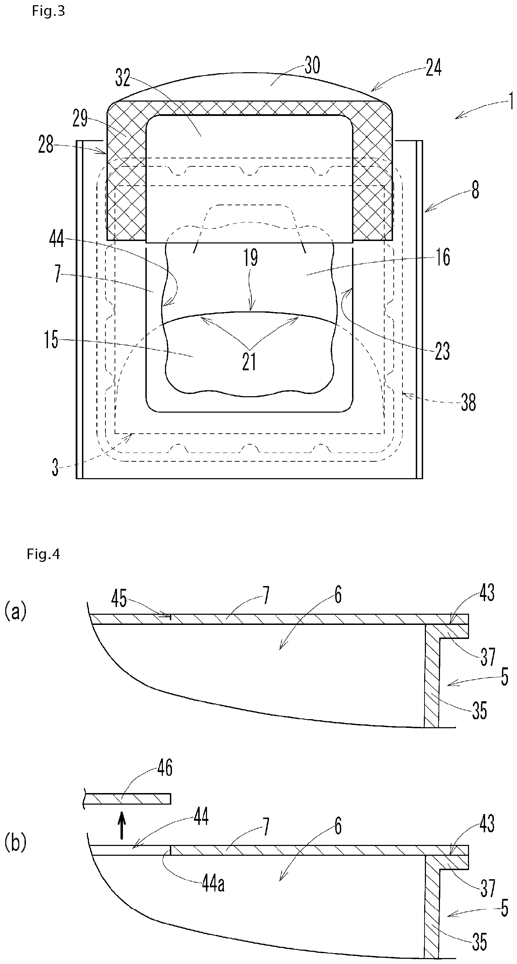

0031 The inner sheet 7 is provided with a breakable part 45 by which can be formed an inner take-out opening 44 that corresponds to the outer take-out opening 23. As shown in Fig. 4(a), the breakable part 45 is formed using a half-cutting method in which a part of the inner sheet 7 is cut out in its thickness direction so that a nonremovable part of the inner sheet 7 in its thickness direction remains. If the inner sheet 7 is pushed at the inside breakable part 45 by a finger, all of the breakable portions of the breakable part 45 are broken, whereby a broken piece 46 is separated from the inner sheet 7 as shown in Fig. 4(b), and the inner take-out opening 44 is formed after the broken piece 46 has been removed. Laser processing is suitably used for performing the half cutting of the inner sheet 7, but other methods may be used.

0032 Also, when the inner sheet 7 is provided with the approximately square-shaped breakable part 45 for the inner take-out opening 44, the breakable part 45 is configured such that, as shown by broken lines in Fig. 5, the four corners of the breakable part 45 bulge outward so that the four corners of the inner take-out opening 44 are provided with recessed areas 45a.

0033 In addition, the entire periphery of the inner sheet 7 may be breakable, so that just pulling a broken piece 38 upwardly enables removal of the entire broken piece 46, or a breakable part 45 may be provided so that the broken piece 46 that remains may be folded under the inner sheet 7.

0034 As shown in Fig. 3, the outer take-out opening 23 of the outer bag 8 and the inner take-out opening 44 of the inner sheet 7 are arranged such that they vertically align with each other. The sizes of the openings of the outer take-out opening 23 and the inner take-out opening 44 are such that a user can insert fingers from his/her left and right hands into the inner container 5, and can grasp the pullable portions 21 of the edge 19 of the topmost facemask 2 of the layered body 3.

0035 Accordingly, in this wet-sheet packaging 1, the layered body 3 that is formed by piling up the folded facemask 18 that has been folded into three parts is bent into an inverted U-shape to be accommodated inside inner container 5, whereby when a user wants to take out the topmost facemask 2, the user grasps two portions of the edge 19 of the upper portion of the facemask 2, whereby the user can spread out the facemask 2 while taking it out.

0036 The inner take-out opening 44 of the inner sheet 7 is slightly smaller than the outer take-out opening 23 of the outer bag 8. That is, the opening edge 44a of the inner take-out opening 44 of the inner sheet 7 is located more inward than is the opening edge 23a of the outer take-out opening 23. As shown in Fig. 8, when a user grasps the center of the topmost facemask 2 of the layered body 3, the outside of the facemask 2 that is being taken out is scraped by the opening edge 44a, so that any excessive amount of the liquid that has been impregnated into the facemask 2 is scraped off. In addition, the periphery of the inner take-out opening 44 is wavy, but the periphery may be straight or circular.

0037 The breakable part 45 is located inside the opening edge 23a of the outer take-out opening 23 of the outer bag 8. Therefore, when the inner take-out opening 44 has been formed by breaking the inner sheet 7 along its breakable part 45, the part of the inner sheet 7 that is the outside of the inner take-out opening 44 corresponds, as a flat surface, to the adhesive part 29 of the closure sheet 28 in a flat surface. Accordingly, when a user pushes the closure sheet 28 from its upper side, the part of the inner sheet 7 that is located outside the inner take-out opening 35 supports the outer surface of the outer bag 8.

0038 The wet-sheet packaging 1 is manufactured in such a way that (1) multiple folded facemasks 18, each of which is formed by folding a wet sheet into three parts, are stacked to form a layered body 3; (2) the layered body 3 is bent in an inverted U-shape so that a layer-top part 26 and layer-side parts 27 are formed; and (3) the layered body 3 is inserted inside the inner container 5 and turned inward so that the two layer-side parts 27 form the bottom of the layered body 3.

0039 Also, when the layered body 3 is inserted inside the inner container 5, as shown in Figs. 1 and 3, the layered body 3 is preferably disposed so that the fold line 13 of the upper portion 15 of the folded facemask 18 faces in the direction of the tab part 30 of the closure sheet 28, and the edge 19 of the upper portion 15 of the folded facemask 18 faces in the direction of the separation-prevention part 31. The layered body 3 may be disposed in directions opposite to the above-specified directions.

0040 The layered body 3 inside the inner container 5 is then pressurized, during which process a prescribed amount of the permeating liquid 4 is injected into the inner container 5. After that, the pressure on the layered body 3 is released. This enables the permeating liquid 4 inside the inner container 5 to easily permeate into each sheet 9 of the facemasks 2 of the layered body 3. Then, after the layered body 3 and the permeating liquid 4 are placed inside the inner container 5, the inner sheet 7 is stuck to the upper peripheral edge 37 of the inner container 5 at the fixed part 43 of the upper peripheral edge 37 to firmly close the opening 6 of the inner container 5, and the inner container 5 is wrapped by the outer bag 8 in that state.

0041 In this wet-sheet packaging 1, the opening 6 of the inner container 5 is firmly closed by the inner sheet 7. Therefore, even if the inner container 5 tilts or falls over, the permeating liquid 4 of the inner container 5 does not leak into the outer bag 8, which makes the wet-sheet packaging 1 easy to handle after the wet-sheet packaging 1 has been manufactured as a product.

0042 Also, this configuration prevents the permeating liquid 4 from leaking out from the inner container 5, allowing the facemasks 2 to constantly remain wet, which allows the amount of the permeating liquid 4 in the inner container 5 to be reduced.

0043 Furthermore, the outer surface of the outer bag 8 of the wet-sheet packaging 1 normally shows a brand name and other relevant information that encourages customers to buy the particular wet-sheet packaging 1. Because the above-described configuration of the wet-sheet packaging 1 prevents the permeating liquid 4 from leaking from the inner container 5, the wet-sheet packaging 1 can be arranged on store's display shelf so that the outer surface of the outer bag 8 faces the customers. Accordingly, this configuration allows the products of this wet-sheet packaging 1 to be displayed on a shelf in a way that is most suitable to motivate customers to buy the displayed products.

0044 In order for a user to take out a facemask 2 from the wet-sheet packaging 1, the user separates the closure sheet 28 from the outer surface of the outer bag 8, and pushes down the center portion of the inner sheet 7, by which the breakable part 45 is broken to open the inner take-out opening 44. Accordingly, this configuration makes it possible (1) to use the inner sheet 7 to firmly close the opening 6 so as to maintain the inner container 5 in a sealed state until the inner take-out opening 44 is made at the inner sheet 7, and (2) to easily form the inner take-out opening 44, whereby the wet sheets inside the inner container 5 can easily be taken out. Also, the broken piece 46 that is created when the inner take-out opening 44 is made at the inner sheet 7 may be pulled out and removed, or may be folded under the inner sheet 7.

0045 After the inner take-out opening 44 has been opened, a user inserts fingers from his/her left and right hands into the inner container 5 through the outer take-out opening 23 and the inner take-out opening 44. Then, as is shown by the fingers and the upper-side 15 depicted by broken lines in Fig. 7, the user grasps the edge 19 of the topmost facemask 2 of the layered body 3 and removes the topmost facemask 2 from the layered body 3 of the facemasks 2. Then, as shown in Fig. 8, the user grasps the two pullable portions 21 with two fingers from each of his/her right and left hands and pulls up the upper portion 15 of the facemask 2.

0046 At this time, the facemasks 2, each of which has been folded into three parts to form folded facemasks 18, are stacked to form the layered body 3, which is bent into an inverted U-shape and accommodated inside the inner container 5. The upper portion 15, the middle portion 16, and the lower portion 17 of the folded facemask 18 of the facemask 2 are layered together in an adhesive manner. Therefore, when the user grasps the two pullable portions 21 with fingers of his/her right and left hands to pull up the facemask 2, the upper portion 15, the middle portion 16, and the lower portion 17 are integrally pulled up so that the folded facemask 18 is separated, as an integral unit, from the upper portion 15 of the facemask 2 that is immediately below the facemask 2 that is being grasped.

0047 Accordingly, the user can spread out a facemask 2 while taking it out from the inner container 5. Therefore, in contrast with the prior-art wet-sheet packaging, which requires that a facemask 2 be taken out from a package in a folded state, and then be spread out for a user to use, the present wet-sheet packaging eliminates the trouble that a user of the prior-art packaging encounters in spreading out the facemask 2. Thus, the present wet-sheet packaging improves a user's convenience of use of facemasks 2.

0048 Also, the facemasks 2 are accommodated inside the inner container 5 in such a way that the facemasks 2, all of which are folded into three parts, are stacked to form a layered body 3 that is bent into an inverted U-shape, so that although the packaging is compact due to its shorter length and width, the time needed for folding each facemask 2 is reduced, and a facemask 2 can easily be spread out by a user when it is taken out from the packaging.

0049 Moreover, the layered body 3 is accommodated inside the inner container 5 in such a way that the folded facemasks 18 of the facemasks 2 are stacked to form a layered body 3 that is bent into an inverted U-shape, and therefore the upper portion 15 of the topmost facemask 2 is near the outer take-out opening 23 and the inner take-out opening 44, so that a user can easily grasp the edge 19 of the facemask 2.

0050 Also, in inserting the layered body 3 into inner container 5, the layered body 3 should be arranged so that the fold line 13 of the upper portion 15 of the folded facemask 18 faces the tab part 30 of the closure sheet 28, and the edge 19 of the upper portion 15 of the folded facemask 18 faces the separation-prevention part 31. In this arrangement, a closure sheet 28 that has been opened does not hinder a user who is trying to take out a facemask 2, and the user can easily grasp and peel off the edge 19 of the upper portion 15 by using his/her finger tips.

0051 In addition, because a facemask 2 is spread out while it is being taken from the inner container 5, as shown in Fig. 8, the facemask 2 is scraped by the opening edge 44a of the inner take-out opening 44 of the inner sheet 7, whereby any excessive amount of the permeating liquid 4 that has permeated into the facemask 2 can be scraped out and removed by the opening edge 44a of the inner sheet 7.

0052 Because of the above features, the present wet-sheet packaging 1 provides the following advantageous effects: (1) no unnecessary amount of the permeating liquid 4 inside the inner container 5 is consumed each time that a facemask 2 is taken out; (2) the amount of the permeating liquid 4 inside the inner container 5 can be maintained at a level that is proportionate to the number of the remaining facemasks 2, and (3) the permeating liquid 4 can be evenly permeated into each of the facemasks 2.

0053 After the facemask 2 has been taken out, the adhesive part 29 of the closure sheet 28 is stuck to the outer surface of the outer bag 8 so as to firmly close the outer take-out opening 23. In addition, because the opening edge 44a of the inner sheet 7 can remove any excessive amount of the permeating liquid 4, and the inner take-out opening 44 is smaller than the outer take-out opening 23, the permeating liquid 4 that has permeated into the facemask 2 hardly sticks to the outer surface of the outer bag 8, whereby the closure sheet 28 can be firmly fixed to the outer surface of the outer bag 8 via the adhesive part 29.

0054 Moreover, when the closure sheet 28 is fixed to the outer surface of the outer bag 8 via the adhesive part 29, the closure sheet 19 is lightly pushed from its upper side by the user's fingers. At this time, because the inner sheet 7 supports the outer bag 8 from its lower side, the closure sheet 28 is firmly fixed to the outer bag 8 via the adhesive part 29. Accordingly, the outer surface of the outer bag 8 is hardly crumpled, so that the outer bag 8 can easily be sealed by the closure sheet 28.

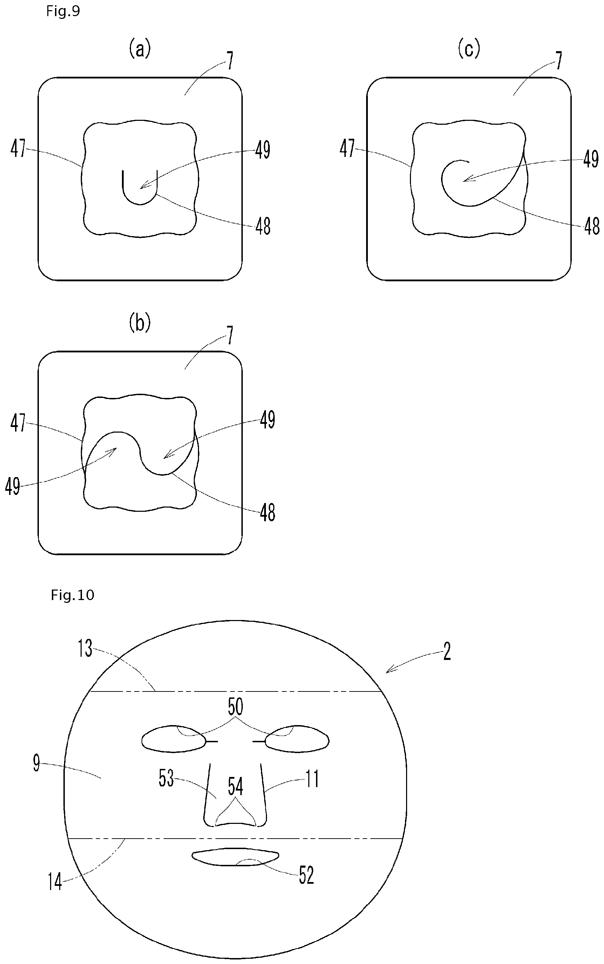

0055 Fig. 9 shows the Second Embodiment of the present invention. A breakable part may be formed along the lines 47, 48, and 49 of an inner sheet 7, as shown in Figs. 9(a) to 9(c). The inner sheet 7 shown in Fig. 9(a) is provided with a first breakable part 47 for an inner take-out opening 44, and a second breakable part 48 that is formed inside the first breakable part 47 in a curved shape such as an approximate U or C shape.

0056 In the example shown in Fig. 9(a), if the center portion of the inner sheet 7 is pushed to break the second breakable part 48, a finger-hooking hole 49 can be formed at the center portion of the inner sheet 7. If a user inserts a finger into the inner sheet 7 through the finger-hooking hole 49, puts the finger at the back of the inner sheet 7, and pulls the inner sheet 7 upward, the first breakable part 47 is broken to form the inner take-out opening 44. Accordingly, this Embodiment allows the user to form the inner take-out opening 44 more easily than does the First Embodiment, and also allows the user to simultaneously remove a broken piece 46.

0057 The inner sheet 7 shown in Fig. 9(b) is provided with a first breakable part 47 for the inner take-out opening 44, and a second breakable part 48 for the finger-hooking hole 49 formed in a curved shape such as an S shape that connects the opposite sides of the first breakable part 47.

0058 In the example shown in Fig. 9(b), the center portion of the inner sheet 7 is pushed to break the second breakable part 48. Then, the user inserts his/her finger under the inner sheet 7 through the broken part, and pulls up the portions along the second breakable part 48, the portions being opposite to each other, by which the inner take-out opening 44 is formed as a result of such portions being broken away along the first breakable part 47. The second breakable part 48 may be configured such that one end or both of its ends are connected to the first breakable part 47 so that a space or spaces exist between the second breakable part 48 and the first breakable part 47.

0059 The inner sheet 7 shown in Fig. 9(c) is provided with a first breakable part 47 for an inner take-out opening 44, and a second breakable part 48 that is shaped like a J and that extends from one side of the first breakable part 47 to the center portion of the inner sheet 7. In the example shown in Fig. 9(c), a user pushes the center portion of the inner sheet 7 to break the second breakable part 48. Then, the user inserts a finger under the inner sheet 7 through the broken part, and pulls the inner-side portion of the second breakable part 47 upward, by which the inner take-out opening 44 is formed as a result of the first breakable part 47 being broken.

0060 Fig. 10 shows the Third Embodiment of the present invention. As shown in Fig. 10, the facemask 2 consists of an approximately circular-shaped sheet 9 that is provided with openings 50 for eyes, a cut-out part 11 for a nose, and an opening 52 for a mouth. The cut-out part for a nose 11 is elongated along both sides to form a nose-covering part 53.

0061 Also, if the cut-out part 11 for a nose is elongated as mentioned above, a breakable connection part 54 may be provided inside the nose-covering part 53, and may be configured such that when a user applies the facemask 2 on his/her face, the user breaks the connection part 54. The other components of this Embodiment are the same as those of the First Embodiment.

0062 As stated above, the facemask 2 can take various forms. The facemask 2, as well as other types of wet sheets, may consist of one sheet 9, or may consist of multiple sheets 9 that are stacked. Therefore, many types of materials may be used for the facemask 2 if the facemask 2 can be folded to form a folded facemask 18.

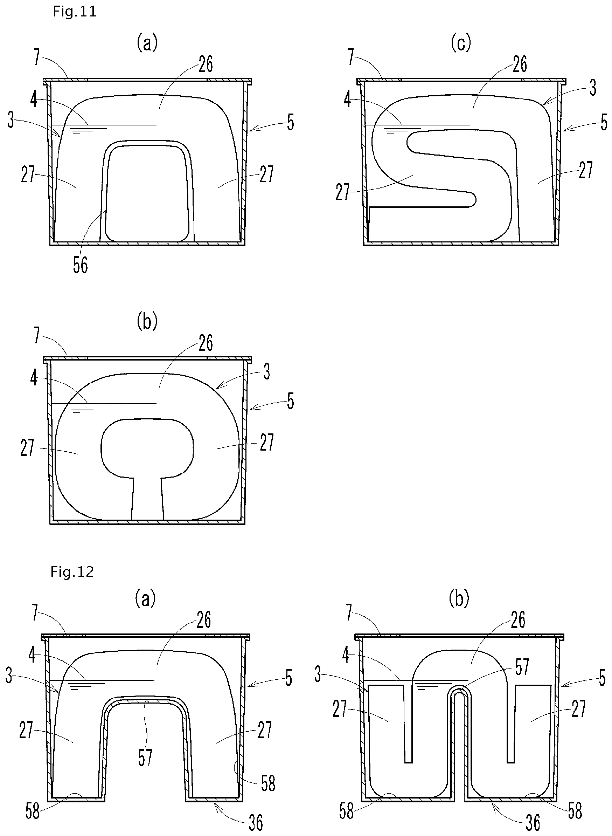

0063 Fig. 11 shows the Fourth Embodiment of the present invention. In accommodating the layered body 3 of the facemasks 2 inside the inner container 5, the folded facemasks 18 that are stacked may generally be bent in any of the ways shown in Figs. 11(a) to 11(c), whereby it is to be noted that solely the solution according to Fig 11(a) corresponds to the inverted U-shape design specified in claim 1.

0064 In Fig. 11(a), a hollow support 56 is disposed inside the inner container 5, and a layered body 3 that is bent to form an inverted U-shape is accommodated inside the inner container 5 such that the layered body 3 straddles the support 56. As stated above, the layered body 3 may be supported by the support 56 inside the inner container 5. In this configuration, because the position of the layer-top part 26 of the layered body 3 is secured by the support 56, a user can easily and securely grasp an edge 19 of the facemask 2. The support 56 is preferably made of a material that is light and not adversely affected by moisture. Also, the support 56 may be omitted in this configuration.

0065 In Fig. 11(b), the layered body 3 is accommodated inside the inner container 5 such that the layered body 3 is bent in an approximately C shape so as to form the layer-top part 26 and layer-side parts 27, whose lower ends are respectively directed to face each other. In this configuration, because the layered body 3 is approximately C-shaped, the height thereof is lower than that of a layered body 3 that is bent in an inverted U-shape, which enables the height of wet-sheet packaging 1 to be lower.

0066 In Fig. 11(c), one layer-side part 27 of the layered body 3 extends approximately straight downward from one side the layer-top part 26, and the other layer-side part 27 extends in an approximately S shape from the other side of the layer-top part 26.

0067 Even if the folded facemask 18 is long, the above configuration allows the layered body 3 to be accommodated inside the inner container 5. Also, in this configuration of the layered body 3, although the layer-top part 26 is not positioned in the central portion of the folded facemask 18 in the longitudinal direction, a user does not experience any problem in spreading out the facemask 2 while taking it out.

0068 In addition, it is preferable that the layered body 3, when accommodating the layered facemasks 2 inside the inner container 5, is appropriately bent or curved so as to be disposed inside the inner container 5 without the layered body 3 needing to be folded in advance at several places in the lengthwise direction. The layered body 3 may be bent or curved into a shape other than an approximate S-shape. The other components of this Embodiment are the same as those of the First Embodiment.

0069 Figs. 12(a) and 12(b) show the present invention's Fifth Embodiment, in which the inner container 5 has a bottom 36 that is provided with a projection 57 by which the layered body 3 is supported, whereby it is to be noted that solely Fig. 12(a) shows the inverted U-shape design of the layered body specified in claim 1.

0070 In Fig. 12(a), a projection 57 is disposed in the center portion of the bottom 36 of the inner vessel 5, and spaces 58 are disposed on both sides of the projection 57. Each space 58 is slightly wider than the thickness of the layered body 3. The layer-top part 26 of the layered body 3 is disposed on top of the projection 57, and two layer-side parts 27 are disposed inside the spaces 58 on opposite sides of the projection 57.

0071 This configuration of the inner container 5 stabilizes the layered body 3 in the inner container 5, which allows a user to easily take out a facemask 2, reduces the amount of permeating liquid 4 required to be poured into the inner container 5, and enhances the rigidity of the bottom 36 of the inner container 5.

0072 In Fig. 12(b), the projection 57 in the center portion of the bottom 36 is small in width, while the spaces 58 on the two sides of the projection 57 are wider than the projection 57. Each space 58 accommodates its respective layer-side part 27 of the layered body 3 such that each of the two layer-side parts 27 are folded into an approximately U shape when inside its respective space 58.

0073 Even if the folded facemask 18 is long, the above configuration allows the layered body 3 to be accommodated inside the inner container 5. The other components of this Embodiment are the same as those of the First Embodiment.

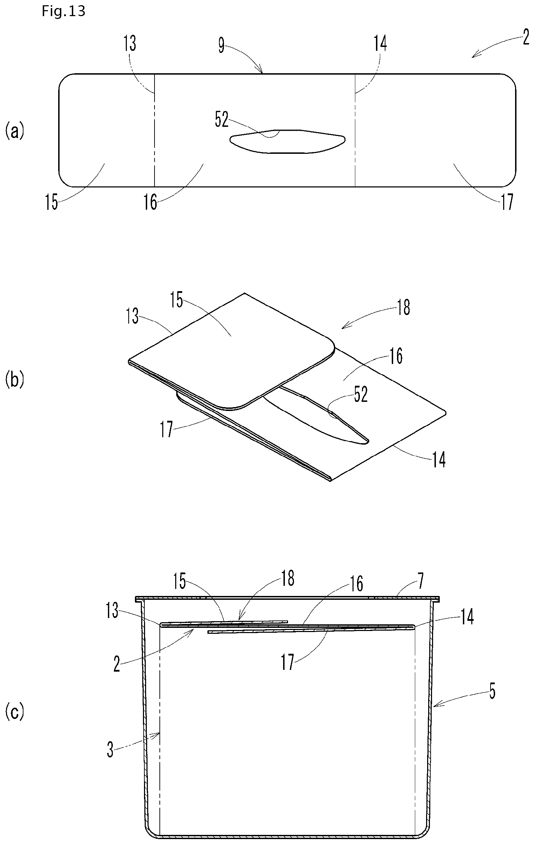

0074 Fig. 13 shows the facemask 2 of the Sixth Embodiment of the present invention. This Embodiment is a facemask 2 that is to be applied to a part of a face, such as the upper half or lower half. For example, in the case of a facemask 2 that is to be applied to the lower half of a face, and that is to be applied to the face from the periphery of the user's mouth to both cheeks of the user, as shown in Fig. 13(a), a long band-shaped sheet 9 is used, which is laterally long when spread out. The sheet 9 is provided with an opening 52 for a mouth.

0075 In this kind of facemask 2, as shown in Fig. 13(b), a folded facemask 18 is configured such that the lengthwise direction of the sheet 9 is folded into three parts along two fold lines 13, 14. Multiple folded facemasks 18 are then stacked to form a layered body 3 that is accommodated inside an inner container 5 as shown in Fig. 13(c). Also, if the sheet 9 is relatively short, it may be folded in the lengthwise direction into two parts along one fold line 13 to form the folded facemask 18. Conversely, if the sheet 9 is long, it may be folded along four or more fold lines to form the folded facemask 18. The other components of this Embodiment are the same as those of the other Embodiments.

0076 In this kind of facemask 2, which uses a band-shaped sheet 9, the folded facemask 18 of the sheet 9 is rectangular or nearly rectangular, as shown in Fig. 13(b). Therefore, in accommodating a layered body 3 of the facemasks 2 inside the inner container 5, it is not necessary to bend the layered body 3 into an inverted U-shape, but it is sufficient that the layered body 3 in which multiple folded facemasks 18 are simply stacked is accommodated inside the inner container 5. In such a case, a user grasps one end of the facemask 2 with two fingers from both his/her right and left hands, whereby the user can spread out the facemask 2 in the lengthwise direction while taking it out. The above accommodation method is also applicable to facemasks 2 that are to be applied to the upper half of a user's face.

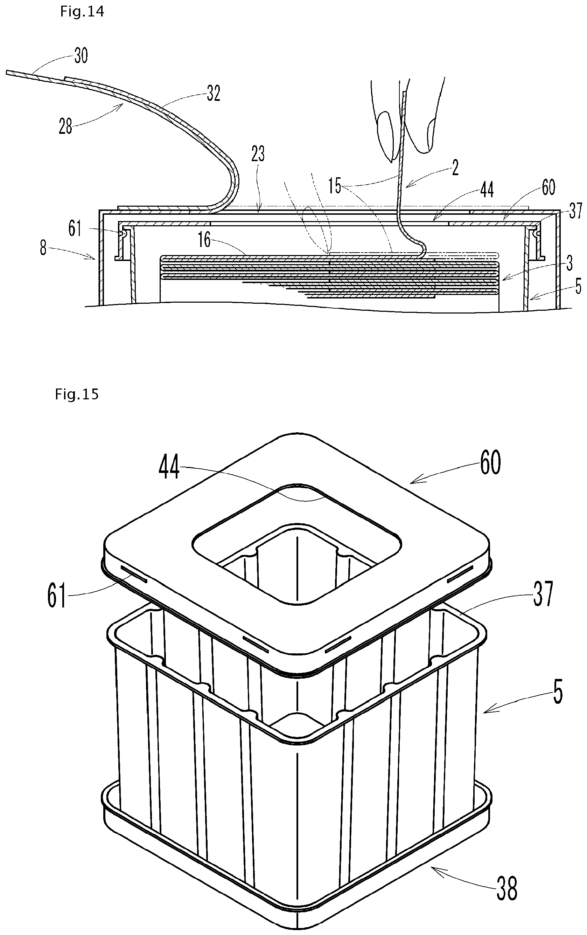

0077 Figs. 14 and 15 show the Seventh Embodiment of the present invention. In this Embodiment, an opening 6 of an inner container 5 is provided with a removable inner lid 60 that has substantially the same shape as a receiving plate 38, and an inner take-out opening 44 through which a facemask 2 is taken out is formed in advance in the center of the inner lid 60. Also, the inner lid 60 has an engagement part 61 that projects inward, and that is removably engaged with the upper peripheral edge 37 of the inner container 5 at the top thereof. The other components of this Embodiment are the same as those of the other Embodiments.

0078 As stated above, the present invention may be configured such that the opening 6 of the inner container 5 is provided with a removable inner lid 60. In such a case, the packaging is made as follows. First a layered body 3 is accommodated inside the inner container 5, into which a permeating liquid 4 is then injected, and the inner lid 60 is placed on the opening 6 of the inner container 5. Last, the inner container 5 is accommodated inside an outer bag 8 to complete the packaging. In addition, because the inner lid 60 has been provided with the inner take-out opening 44, the inner container 5 must be dealt with carefully during the above packaging process, so that the impregnating liquid 4 is not leaked from the inner container 5.

0079 The Embodiments of the present invention have been described above in detail, but the present invention is not limited to such Embodiments, and various modifications thereto can be made. For example, the Embodiments are configured to include a receiving plate 38 that is located below the inner container 5 and that receives the inner container 5, but such a receiving plate 38 may be omitted.

0080 The breakable part 45 is generally sufficiently constituted by one continuous or dotted line, but multiple breakable parts 45 may be provided so that a user can select a preferred size of the breakable part 45 for forming the inner take-out opening 44, whereby the size of the inner take-out opening 35 can be adjusted according to the user's preference.

0081 In order to ensure that the inner sheet 7 firmly closes the opening 6 of the inner container 5, a support frame to which the inner sheet 7 is provided may be attached to the opening 6 of the inner container 5 for firmly closing the opening 6. General bonding methods including heat welding may be used for fixing the inner sheet 7 to the inner container 5.

0082 As a material for the inner sheet 7, a composite film that has gas-barrier capability is suitable, but because the inner container 5 is firmly wrapped by the outer bag 8, if the material for the inner sheet 7 is able to prevent leaking of the liquid, the material does not necessarily require gas-barrier capability.

0083 The adhesive part 29 of the closure sheet 28 is provided as a wide piece to the outer periphery of the outer take-out opening 23, but multiple line-shaped adhesive parts 29 extending outward may instead be provided.

0084 The size of the inner take-out opening 44 is appropriately determined by balancing (1) a user's ease of use when inserting his/her fingers into the inner take-out opening 44 to grasp a facemask 2, and (2) the effectiveness of the inner take-out opening 44 in removing any excess liquid contained in a facemask 2 when the facemask 2 is taken out from the inner take-out opening 44.

0085 The amount of the permeating liquid 4 that can be scraped out by the opening edge 44a of the inner take-out opening 44 is determined by the relative dimensions of the facemask 2 and those of the inner take-out opening 44. Therefore, the size of the inner take-out opening 44 may be configured such that, depending on the direction in which the facemask 2 is drawn out, the facemask 2 may lightly contact the opening edge 44a of the inner take-out opening 44, or the permeating liquid 4 might not be scraped out by the opening edge 44a.

0086 It is preferable that the entire periphery of the opening edge 44a of the inner take-out opening 44 is located inside the opening edge 23a of the outer take-out opening 23. Also, it is preferable that a predetermined amount of space is provided between the opening edge 44a of the inner take-out opening 44 and the opening edge 23a of the outer take-out opening 23 so that the impregnating liquid 4 of the facemask 2 will not adhere to the adhesive part 29. However, if there is sufficient space between the inner periphery of the adhesive part 29 and the opening edge 23a of the outer take-out opening 23, the opening edge 44a of the inner take-out opening 44 may be located at the approximately same position as the opening edge 44a of the outer take-out opening 44, or the opening edge 44a of the inner take-out opening 44 may be located slightly inside the opening edge 23a of the outer take-out opening 23.

0087 In forming the folded facemask 18 using a wet sheet such as the facemask 2, it is common that the sheet 9 is folded into three parts, but the sheet 9 may be folded into four or more parts.

0088 In the above Embodiments, the closure means 24 of the outer bag 8 is configured such that the closure sheet 28 is separably stuck to the outer surface of the outer bag 8. However, the closure means may include both an opening frame that surrounds the outer take-out opening 23 of the outer bag 8 and an openable lid connected to the opening frame via a hinge. The openable lid may be detachably provided to the opening frame.

### Descriptions of Reference Numbers

0089
- 1: wet-sheet packaging
- 2: facemask (wet sheet)
- 3: layered body
- 5: inner container
- 6: opening
- 7: inner sheet
- 8: outer bag
- 13, 14: fold lines
- 18: folded facemask
- 19: edge
- 21: pullable portion
- 23: outer take-out opening
- 24: closure means
- 26: layer-top part
- 27: layer-side parts
- 35: inner take-out opening
- 57: projection
- 58: space

## Claims

1. A wet-sheet packaging (1) comprising an outer bag (8) that includes an outer take-out opening (23) on the bag's upper side, the outer take-out opening being freely opened and closed by a closure means (24); and a layered body (3) of wet sheets (2), each of the wet sheets (2) being in a folded state, the outer bag (8) accommodating the layered body (3) of the wet sheets (2), wherein the layered body (3) is accommodated in a way that allows an edge (19) of the upper surface-side of the topmost wet sheet (2) to be grasped by a user and spread out when the wet sheet (2) is taken out of the packaging (1),
each of the wet sheets (2) is folded into a band shape to be layered such that each of the wet sheets (2) is folded at fold lines (13, 14) that are substantially parallel to each other into a zigzag shape, and the edge (19) is configured to be opposite the outer take-out opening (23), and to be disposed at the middle part of the folded sheet in the width direction so as to face the lengthwise direction,
**characterized in that** the layered body (3) includes a layer-top part (26) that faces the outer take-out opening (23), and layer-side parts (27) that are bent in their lengthwise direction into an approximately inverted U-shape, and that stretch from both sides of the layer-top part (26) to each of the bottom edges of the layered body (3).

2. The wet-sheet packaging of Claim 1, wherein the outer bag (8) includes an inner container (5) that has shape-retention capability and that accommodates the layered body (3).

3. The wet-sheet packaging of Claim 2, further comprising an inner sheet (7) by which an opening (6) of the inner container (5) is firmly closed and within which an inner take-out opening (44) corresponding to the outer take-out opening (23) is to be formed.

4. The wet-sheet packaging of Claim 2 or Claim 3, wherein the inner container (5) includes a projection (57) that protrudes from the bottom (36) of the container to support the underside of the layer-top part (26) that faces the outer take-out opening (23) of the layered body (3), and recessed parts (58) formed on both sides of the projection (57), with said recessed parts (58) accommodating the layer-side parts (27) that respectively stretch from the layer-top part (26) to the bottom edges of the layered body (3).

5. The wet-sheet packaging of any of Claims 1 to 4, wherein the outer take-out opening (23) is a size that allows a user to insert two fingers from both his/her left and right hands into the opening (23) and to grasp two portions of the edge (19).

6. The wet-sheet packaging of any of Claims 1 to 5, wherein the wet sheet (2) is a facemask (2) that is used for face packing, and that is permeated with a cosmetic liquid.

7. The wet-sheet packaging of Claim 6, wherein the facemask (2) is folded in reverse directions at multiple fold lines (13, 14) disposed in the lengthwise direction, with the edge (19) being at the outer periphery (37) of the upper side of the facemask (2).

## Patentansprüche

1. Verpackung für Feuchttücher (1), umfassend einen äußeren Beutel (8), der eine äußere Entnahmeöffnung (23) auf der oberen Seite des Beutels beinhaltet, wobei die äußere Entnahmeöffnung durch ein Verschlussmittel (24) frei geöffnet und geschlossen wird; und einen geschichteten Körper (3) von Feuchttüchern (2), wobei sich jedes der Feuchttücher (2) in einem gefalteten Zustand befindet, wobei der äußere Beutel (8) den geschichteten Körper (3) der Feuchttücher (2) aufnimmt, wobei der geschichtete Körper (3) auf eine Weise aufgenommen ist, die ermöglicht, dass eine Kante (19) der oberen Flächenseite des obersten Feuchttuchs (2) von einem Benutzer gegriffen und ausgebreitet wird, wenn das Feuchttuch (2) aus der Verpackung (1) genommen wird,
jedes der Feuchttücher (2) in eine Bandform gefaltet ist, um derart geschichtet zu sein, dass jedes der Feuchttücher (2) an Falzlinien (13, 14), die im Wesentlichen parallel zueinander sind, in einer Zigzagform gefaltet sind, und die Kante (19) konfiguriert ist, um sich gegenüber der äußeren Entnahmeöffnung (23) zu befinden, und um am mittleren Teil des gefalteten Tuchs in der Breitenrichtung angeordnet zu sein, um auf die Längsrichtung zu zeigen,
**dadurch gekennzeichnet, dass** der geschichtete Körper (3) einen Schichtoberteil (26) umfasst, der auf die äußere Entnahmeöffnung (23) zeigt, und Schichtseitenteile (27), die in ihrer Längsrichtung in eine annähernd umgekehrte U-Form gebogen sind, und die sich von beiden Seiten des Schichtoberteils (26) in jede der Bodenkanten des geschichteten Körpers (3) erstrecken.

2. Verpackung für Feuchttücher nach Anspruch 1, wobei der äußere Beutel (8) einen inneren Behälter (5) beinhaltet, der eine Fähigkeit zur Formbeständigkeit aufweist, und der den geschichteten Körper (3) aufnimmt.

3. Verpackung für Feuchttücher nach Anspruch 2, weiter umfassend ein inneres Tuch (7), durch das eine Öffnung (6) des inneren Behälters (5) fest geschlossen ist, und worin eine innere Entnahmeöffnung (44), die der äußeren Entnahmeöffnung (23) entspricht, gebildet werden soll.

4. Verpackung für Feuchttücher nach Anspruch 2 oder Anspruch 3, wobei der innere Behälter (5), einen Vorsprung (57) beinhaltet, der vom Boden (36) des Behälters vorspringt, um die Unterseite des Schichtoberteils (26) zu stützen, der auf die äußere Entnahmeöffnung (23) des geschichteten Körpers (3) zeigt, und vertiefte Teile (58), die auf beiden Seiten des Vorsprungs (57) gebildet sind, wobei die vertieften Teile (58) die Schichtseitenteile (27) aufnehmen, die sich jeweils vom Schichtoberteil (26) zu den Bodenkanten des geschichteten Körpers (3) erstrecken.

5. Verpackung für Feuchttücher nach einem der Ansprüche 1 bis 4, wobei die äußere Entnahmeöffnung (23) eine Größe aufweist, die es einem Benutzer/einer Benutzerin ermöglicht, zwei Finger von sowohl seiner/ihrer linken als auch rechten Hand in die Öffnung (23) einzuführen und zwei Abschnitte der Kante (19) zu greifen.

6. Verpackung für Feuchttücher nach einem der Ansprüche 1 bis 5, wobei das Feuchttuch (2) eine Gesichtsmaske (2) ist, die verwendet wird, um das Gesicht abzudecken, und die mit einer kosmetischen Flüssigkeit durchtränkt ist.

7. Verpackung für Feuchttücher nach Anspruch 6, wobei die Gesichtsmaske (2) in umgekehrten Richtungen an zahlreichen Falzlinien (13, 14) gefaltet ist, die in der Längsrichtung angeordnet sind, wobei sich die Kante (19) am äußeren Umfang (37) der oberen Seite der Gesichtsmaske (2) befindet.

## Revendications

1. Emballage pour feuilles humides (1) comprenant un sachet externe (8) qui comprend une ouverture de retrait externe (23) sur le côté supérieur du sachet, l'ouverture de retrait externe étant librement ouverte et fermée par un moyen de fermeture (24) ; et un corps stratifié (3) de feuilles humides (2), chacune des feuilles humides (2) étant dans un état plié, le sachet externe (8) logeant le corps stratifié (3) de feuilles humides (2), dans lequel le corps stratifié (3) est logé d'une manière qui permet à un utilisateur de saisir et d'étaler un bord (19) du côté de la surface supérieure de la feuille humide la plus haute (2) lorsque la feuille humide (2) est retirée de l'emballage (1),
chacune des feuilles humides (2) est pliée en une forme de bande pour être stratifiée de sorte que chacune des feuilles humides (2) est pliée selon des lignes de pliage (13, 14) qui sont sensiblement parallèles entre elles en une forme de zigzag, et le bord (19) est configuré pour être opposé à l'ouverture de retrait externe (23) et pour être disposé au niveau de la partie centrale de la feuille pliée dans le sens de la largeur afin de faire face à la direction de longueur,
**caractérisé en ce que** le corps stratifié (3) comprend une partie de strate supérieure (26) qui fait face à l'ouverture de retrait externe (23), et des parties de strate latérale (27) qui sont pliées dans le sens de leur longueur en une forme approximative de U inversé et qui s'étirent à partir des deux côtés de la partie de strate supérieure (26) vers chacun des bords inférieurs du corps stratifié (3).

2. Emballage pour feuilles humides selon la revendication 1, dans lequel le sachet externe (8) comprend un récipient interne (5) qui a une capacité de retenue par sa forme et qui loge le corps stratifié (3).

3. Emballage pour feuilles humides selon la revendication 2, comprenant en outre une feuille interne (7) grâce à laquelle une ouverture (6) du récipient interne (5) est fermement fermée et à l'intérieur de laquelle une ouverture de retrait interne (44) correspondant à l'ouverture de retrait externe (23) doit être formée.

4. Emballage pour feuilles humides selon la revendication 2 ou la revendication 3, dans lequel le récipient interne (5) comprend une saillie (57) qui fait saillie du fond (36) du récipient pour supporter la face inférieure de la partie de strate supérieure (26) qui fait face à l'ouverture de retrait externe (23) du corps stratifié (3), et des parties évidées (58) formées sur les deux côtés de la saillie (57), avec lesdites parties évidées (58) qui logent les parties de strate latérale (27) qui s'étirent respectivement à partir de la partie de strate supérieure (26) jusqu'aux bords inférieurs du corps stratifié (3).

5. Emballage pour feuilles humides selon l'une quelconque des revendications 1 à 4, dans lequel l'ouverture de retrait externe (23) est une taille qui permet à un utilisateur d'insérer deux doigts de ses mains gauche et droite dans l'ouverture (23) et de saisir deux parties du bord (19).

6. Emballage pour feuilles humides selon l'une quelconque des revendications 1 à 5, dans lequel la feuille humide (2) est un masque pour le visage (2) qui est utilisé pour être mis en place sur le visage, et qui est imprégné avec un liquide cosmétique.

7. Emballage pour feuilles humides selon la revendication 6, dans lequel le masque pour le visage (2) est plié dans des directions inverses au niveau de plusieurs lignes de pliage (13, 14) disposées dans le sens de la longueur, avec le bord (19) qui est au niveau de la périphérie externe (37) du côté supérieur du masque pour le visage (2).
